# EUROPÄISCHE PATENTSCHRIFT

(11) **EP 1 316 324 B1**
(45) Veröffentlichungstag und Bekanntmachung des Hinweises auf die Patenterteilung: **10.08.2005**
(21) Anmeldenummer: 01128614.3
(22) Anmeldetag: 30.11.2001
(51) Int. Cl.: A61M 5/14, A61M 5/172, A61B 5/055, G06F 19/00

(54) **Vorrichtung zur Planung einer Infusion**
Device for planing an infusion
Dispositif pour planifier une infusion

(43) Veröffentlichungstag der Anmeldung: 04.06.2003
(73) Patentinhaber: BrainLAB AG, 85551 Kirchheim/Heimstetten (DE)
(72) Erfinder: Tanner, Philipp, Dr., 80769 München (DE); Hartlep, Andreas, Dr., 80803 München (DE); Pedain, Christoph, 81667 München (DE)
(74) Vertreter: Schwabe - Sandmair - Marx

(56) Entgegenhaltungen:
- WO-A-01/85230
- US-A- 5 919 135
- US-B1- 6 293 282

## Beschreibung

Die vorliegende Erfindung bezieht sich auf Verfahren, Vorrichtungen und Computerprogramme, welche zur Vorbereitung, Planung und Durchführung einer Infusion verwendet werden.

Allgemein soll erfindungsgemäß unter dem Begriff "Infusion" jede Verabreichung einer zum Beispiel flüssigen oder festen Substanz bzw. eines Infusionsmittels, wie zum Beispiel Medikamente, Zellen, Gene, Enzyme, Proteine, Antikörper, Hormone, Viren oder ähnliches verstanden werden, wobei diese Substanz direkt in einen Körper bzw. in Körpergewebe eingebracht wird, um zum Beispiel die Blut-Hirn-Schranke zu überwinden. Das Zuführen der Substanz kann innerhalb relativ kurzer Zeit zum Beispiel durch eine Injektion oder über einen längeren Zeitraum , beispielsweise bei kontinuierlicher und gegebenenfalls variabler Zufuhrrate der Substanz erfolgen.

Die Verabreichung von pharmazeutischen Substanzen erfolgte früher durch das Einspritzen einer Substanz durch die Haut, direkt in das Gefäßsystem, Muskel- oder Unterhautgewebe oder das Setzen eines Katheters, so dass eine Substanz von außen unmittelbar in Körpergewebe eingebracht werden konnte. Dabei kam es entscheidend auf die Erfahrung der Person an, welche zum Beispiel die Spritze ansetzte, um eine zu verabreichende Substanz möglichst genau im gewünschten Gewebebereich positionieren zu können. Aufgrund von Ungenauigkeiten und der Unkenntnis der substanz- und patientenspezifischen Ausbreitungs- und Verteilungsmechanismen im entsprechenden Gewebe sowie aufgrund von patientenspezifischen Verschiedenheiten der Gewebeanordnung, zum Beispiel im Falle von Gewebeerkrankungen, war es erforderlich, eine für ein bestimmtes Zielgewebe zur Behandlung erforderliche Dosis relativ hoch anzusetzen, um sicherzustellen, dass die zugeführte Substanz mit ausreichend hoher Dosis in das Zielgewebe gelangen kann, selbst wenn der Einbringungsort der Substanz aufgrund von Ungenauigkeiten nicht unmittelbar im Zielgewebe liegt. Jedoch sind solche Substanzen zum Beispiel zur Tumor-Behandlung oft toxisch und belasten somit unnötig umliegendes Gewebe. Soll zum Beispiel ein Tumor durch eine Chemotherapie behandelt werden, so werden toxische Substanzen eingesetzt, welche auch Schaden in gesundem Gewebe anrichten können.

Andererseits muss bei einer Infusion sichergestellt werden, dass die Infusionssorte und -parameter die komplette Behandlung des Infusionszielgebietes ermöglichen.

Um die Einbringung einer Substanz in einen bestimmten Gewebebereich überhaupt zu ermöglichen oder effizienter zu gestalten um zum Beispiel Tumore, die Parkinsonsche Krankheit oder andere Erkrankungen zu behandeln, wurden bereits verschiedene Verfahren und Vorrichtungen vorgeschlagen.

Aus der US RE 37,410 E ist es bekannt eine zu verabreichende Substanz in ein biologisch abbaubares Material einzubringen und dieses innerhalb oder eng benachbart zu einem zu behandelnden Tumor anzuordnen, um so zum Beispiel im Falle eines Gehirntumors die Blut-Hirn-Schranke zu überwinden. Nach dem Abbauen des biologisch abbaubaren Materials wird die darin enthaltene Substanz abgegeben. Jedoch ist dieses Verfahren relativ ungenau bezüglich der patientenspezifischen individuellen Dosierung und es kann auch nicht für eine länger andauernde spezifische Behandlung verwendet werden, bei welcher eventuell Behandlungsparameter geändert werden sollen.

Aus der US 5,720,720 ist ein Verfahren zum Durchführen von Mikroinfusionen bekannt, wobei die Spitze eines Infusionskatheters innerhalb einer Gewebestruktur angeordnet wird. Ein Wirkstoff wird durch den Katheter zugeführt, wobei ein Druck-Gradient an der Spitze des Katheters während der Infusion konstant gehalten wird. Dadurch soll eine bessere Verteilung des Wirkstoffes sichergestellt werden, welche durch die Fusion allein nicht erreichbar wäre.

Die US 6,026,316 beschreibt ein Verfahren zur Zufuhr von Medikamenten, wobei durch Kernspinresonanz (MR) gewonnene Daten verwendet werden, um die Position der Zuführvorrichtung zu ermitteln und die räumliche Verteilung des zugeführten Medikamentes zu überwachen.

Aus der US 5,919,135 sind ein System und ein Verfahren zur Behandlung von Tumoren durch Infusion von Medikamenten bekannt. Insbesondere soll die Zufuhr von Medikamenten in Echtzeit durch eine interaktive Computersteuerung überwacht werden.

Die US 6,293,282 B1 offenbart die Verwendung eines Computer-Abbildungssystems, um die Position eines Endoskops innerhalb des Körpers eines Patienten zu erfassen.

Aus der WO 01/85230 A2 ist das Modellieren der Bewegung von Material in einem Organismus durch ein gleichförmiges strukturiertes Feld aus statischen Konstanten, welche den Transport bestimmen, bekannt. Es ist eine Aufgabe der vorliegenden Erfindung Verfahren, Vorrichtungen und Software vorzuschlagen, mit welchen eine zu verabreichende Substanz bzw. ein Wirkstoff patientenspezifisch dosiert in einen definierten Gewebebereich eingebracht werden kann.

Diese Aufgabe wird durch die Gegenstände der unabhängigen Ansprüche gelöst. Vorteilhafte Ausführungsformen ergeben sich aus den Unteransprüchen.

Gemäß einem ersten Aspekt bezieht sich die Erfindung auf ein Verfahren zur Planung einer Infusion, d. h. zur Verabreichung einer Substanz oder eines Wirkstoffes, insbesondere zur Einbringung in Gewebe, bevorzugt in eine vorgegebene Gewebestruktur oder ein Gewebevolumen, wobei Patientendaten oder daraus gewonnene Patientenparameter erfasst werden. Hierzu können bekannte Kernspinresonanzverfahren (MRI), Computertomographie (CT)-Verfahren, Röntgen-, Ultraschall-Verfahren oder andere geeignete Verfahren verwendet werden, welche es ermöglichen, die räumliche Struktur eines Körpers, insbesondere der Gewebestruktur zu erfassen und darzustellen und/oder funktionelle Daten wie zum Beispiel patientenspezifische Diffusions- und Perfusionseigenschaften zu gewinnen. Unter Verwendung der ermittelten Patientendaten wird erfindungsgemäß die Durchführung der Infusion geplant, d. h. es werden unter Berücksichtigung verschiedener auswählbarer Vorgaben, wie zum Beispiel den Patientendaten oder auch Parametern von zur Verfügung stehenden zu verabreichenden Substanzen, Parametern der verwendbaren Katheter und gegebenenfalls einer verwendbaren Pumpe, beispielsweise eine geeignete Auswahl des zu verwendenden Katheters getroffen, die Positionierung des Katheters hinsichtlich Anbringungsort und Eindringtiefe vorgenommen, das Infusionsmittel ausgewählt und falls erforderlich modifiziert, zum Beispiel verdünnt, und der zeitliche Verlauf des Druckes, mit welchem das Infusionsmittel durch den Katheter zugeführt werden soll, vorgegeben. Ziel einer solchen Auswahl und Einstellung ist es, eine definierte Menge der zu verabreichenden Substanz in ein Zielgewebevolumen einzubringen, um dort eine bestimmte Konzentration zu erhalten, wobei so wenig der zu verabreichenden Substanz wie möglich in Nicht-Zielgewebe eingebracht werden soll.

Zur Positionierung der Infusionsvorrichtung(en), beispielsweise eines oder mehrerer Katheter, können die erfassten Patientendaten verwendet werden, wobei aus diesen Patientendaten ermittelt wird, wo genau sich im Körper des Patienten ein zu behandelndes Gewebevolumen, wie zum Beispiel ein Gehirntumor befindet. Unter Verwendung dieser Information kann ein geeigneter Katheter zum Beispiel aus einer vorhandenen Datenbank durch einen Benutzer oder vollautomatisch ausgewählt werden und gegebenenfalls durch Nachbearbeitung, zum Beispiel zurechtschneiden der Katheterlänge, entsprechend anwendungs- oder patientenspezifisch zum Beispiel bezüglich der gewünschten Eindringtiefe in Gewebe modifiziert werden. Weiterhin kann ermittelt werden, wo eine geeignete Stelle zum Anbringen des Katheters ist, um möglichst wenig gesundes Gewebe durch die Infusion zu belasten.

Vorteilhaft können bekannte Verfahren zur Positionierung zum Beispiel unter Verwendung von reflektierenden Markern, welche am Katheter angebracht werden und von IR-Kameras detektiert werden, verwendet werden, um den Katheter an einer gewünschten Position des Patienten anzubringen. Dabei können am Patienten selbst ebenfalls Marker angebracht sein, welche als Referenz dienen und durch welche ein Patientenkoordinatensystem festgelegt wird, in welchem der Katheter an einer bestimmten ermittelten Stelle platziert wird.

Bevorzugt werden unter Verwendung der erfassten Patientendaten zur Planung der Infusion patientenspezifische Parameter ermittelt, wie zum Beispiel die Gewebe- oder Körperstruktur im Bereich des durch eine Infusion zu behandelnden Gewebes. Insbesondere ist es vorteilhaft, die Gewebedichte, die Verteilung bestimmter Gewebestrukturen, oder die Durchblutung eines bestimmten Gewebebereiches als Patientenparameter zu ermitteln. Patientenparameter können sowohl unmittelbar aus den erfassten Patientendaten, als auch aus Datenbanken oder einer Kombination aus in Datenbanken gespeicherten Werten zusammen mit den erfassten Patientendaten gewonnen werden. So können beispielsweise in Datenbanken Werte bezüglich der gewöhnlichen Durchblutung bestimmter Gewebebereiche, das Diffusions- und Perfusionsverhalten ausgewählter Substanzen in dem betrachteten Gewebe, Werte bezüglich des Gewebeverhaltens nach Zufuhr einer bekannten Substanz, zum Beispiel durch Anschwellen des Gewebes oder Stoffwechselreaktionen gespeichert werden, welche als Patientenparameter zur Planung einer Infusion verwendet werden können.

Es ist weiterhin vorteilhaft, Infusionsmittelparameter zu ermitteln, welche charakteristisch für die zu verabreichende Substanz oder einen Wirkstoff sind und beispielsweise die physikalischen, chemischen und/oder biologischen Eigenschaften definieren. So kann beispielsweise aus einer Datenbank eine Information bezüglich der Molekül- oder Teilchengröße der zu verabreichenden Substanz, der Diffusionsgeschwindigkeit dieser Substanz in einer bestimmten Gewebeart, den Metabolismus bzw. die Wechselwirkung der Substanz mit Gewebe aufgrund von Stoffwechselprozessen, ein für die Substanz für den zu behandelnden Gewebetyp bekannten Diffusionskoeffizienten oder vorteilhaften Injektionsdruck oder Druck-Gradienten, eine vorteilhafte Konzentration, Menge oder Zufuhrrate, deren Größenordnung gewöhnlich im Bereich ml/h liegt, gewonnen werden. Die beispielhaft aufgezählten Infusionsmittelparameter können einzeln oder in Kombination zusammen mit anderen Parametern zur Planung der Infusion verwendet werden.

Vorteilhaft werden Katheterparameter, d. h. für einen Katheter spezifische Größen zur Planung der Infusion verwendet, wobei verschiedene Kathetertypen zum Beispiel in einer Datenbank zur Auswahl zur Verfügung gestellt werden können. Für die Infusion relevante Katheterparameter können zum Beispiel der Innendurchmesser des Katheters, Oberflächenbeschaffenheit, das Material, insbesondere die Steifigkeit des Katheters, die Anzahl und Anordnung der Austrittsöffnungen an dem Katheter oder eine bekannte Eignung eines bestimmten Kathetertyps für eine bestimmte zu verabreichende Substanz oder einen bestimmten zu behandelnden Gewebetyp oder Gewebeerkrankung sein. Allgemein können erfindungsgemäß auch mehrere Katheter verwendet werden.

Als Infusionsmittel kann erfindungsgemäß beispielsweise ein Medikament, eine Lösung mit Zellen, Viren, Genen, Enzymen, Proteinen, Hormonen, Antikörpern oder einer Kombination daraus verwendet werden.

Unter Verwendung der oben beispielhaft aufgeführten Patientenparameter, Infusionsmittelparameter und/oder Katheterparameter einzeln oder in Kombination, zusammen mit den erfassten Patientendaten kann eine durchzuführende Infusion erfindungsgemäß so geplant werden, dass ein möglichst großer Anteil einer Substanz in einen Zielgewebebereich durch Infusion eingebracht wird, wobei möglichst wenig der Substanz in Nicht-Zielgewebe abgegeben wird. Eine durch Infusion in Gewebe einzubringende Substanz kann erfindungsgemäß somit mit einem besonders geeigneten und richtig positionierten Kathetertyp und dem richtigen Injektionsdruck in einer geeigneten Konzentration mit einer gewünschten Rate unter Berücksichtigung von Stoffwechsel- und Diffusionsprozessen in einen zu behandelnden Gewebebereich eines Patienten eingebracht werden, um in diesem Gewebebereich eine gewünschte Konzentration der einzubringenden Substanz zu erhalten, wobei beispielsweise im Fall von Gehirntumoren die Blut-Hirn-Schranke durch das direkte Einbringen der Substanz durch Infusion überwunden werden kann. Umliegendes Gewebe wird dabei so wenig wie möglich belastet.

Vorteilhaft kann erfindungsgemäß zur Vorwärts-Planung der Infusion eine Simulation der durchzuführenden Infusion, zum Beispiel durch Berechnung der Infusionsmittelverteilung in Gewebe unter Verwendung der erfassten Patientendaten und der oben genannten verschiedenen Parameter durchgeführt werden. Durch eine solche Simulation kann die Infusionsmittelverteilung sowohl statisch als auch dynamisch als Funktion der Zeit ermittelt und vorteilhaft graphisch dargestellt werden. Somit kann bereits vor Durchführung einer Infusion festgestellt werden, ob eine gewünschte Konzentrationsverteilung der einzubringenden Substanz in dem Zielgewebe erhalten werden kann, oder ob gegebenenfalls Infusionsmittelparameter, Katheterparameter oder Positionsparameter verändert werden müssen, um einen besseren Erfolg der Infusion sicherzustellen.

Weiterhin kann erfindungsgemäß auch eine Rückwärts- oder inverse Planung erfolgen, wobei zum Beispiel von einer Bedienperson bestimmte Behandlungsdaten vorgegeben werden, wie zum Beispiel das zu behandelnde Zielvolumen, vorteilhaft zusammen mit Risikostrukturen wie zum Beispiel Nervenbahnen, welche nicht durch die Infusion beeinträchtigt werden sollten und die Angabe des zu behandelnden Gewebetyps. Dabei kann entweder vollautomatisch oder in Wechselwirkung mit dem Benutzer, zum Beispiel durch Anzeige eines Auswahlmenüs, der Ablauf der Infusion festgelegt werden, d. h. es können ein oder mehrere Kathetertypen zusammen mit geeigneten Infusionsmitteln ausgewählt, die Katheteranordnung(en) hinsichtlich Position und/oder Eindringtiefe festgelegt und die Infusionsmittelparameter eingestellt werden, um eine möglichst optimale Infusionsbehandlung für das vorgegebene Zielvolumen zu ermöglichen.

Die oben beschriebenen Planungsverfahren, insbesondere die Auswahl der einzelnen Parameter, können vollautomatisch zum Beispiel unter Verwendung von in Datenbanken gespeicherten Werten, halbautomatisch zum Beispiel durch noch von einem Benutzer zu treffende Auswahlen aus einem angezeigten Menü oder manuell zum Beispiel durch von einem Benutzer einzugebende Parameterwerte erfolgen. Vorteilhaft werden hierbei geeignete Rechner zusammen mit Eingabe- und Ausgabe-, zum Beispiel Anzeigeelementen verwendet, welche auszuwählende Elemente, Gewebestrukturen, berechnete Konzentrationsverteilungen des Infusionsmittels im Gewebe und weitere Informationen anzeigen.

Gemäß einem weiteren Aspekt bezieht sich die vorliegende Erfindung auf ein Computerprogramm, welches, wenn es in einen Computer geladen ist oder auf einem Computer läuft, das oben beschriebene Verfahren oder Teile davon ausführt. Ebenso bezieht sich die vorliegende Erfindung auf ein Speichermedium für ein solches Programm oder ein Computerprogrammprodukt mit dem oben erwähnten Programm.

Eine erfindungsgemäße Vorrichtung zur Planung einer Infusion weist ein Planungssystem auf, welches aus einem Computersystem, bevorzugt mit Eingabe- und Ausgabevorrichtungen und zugehöriger Software besteht. Dabei ist vorteilhaft ein Monitor zur Anzeige von durch den Computer aus Datenbanken vorgegebenen Elementen oder aus Berechnung ermittelten Werten oder räumlichen Verteilungen vorgesehen.

Vorteilhaft ist auch ein Navigationssystem vorgesehen, welches zum Beispiel aus an zu positionierenden Elementen angebrachten reflektierenden Markern, Leuchtdioden oder Spulen und IR-Kameras oder Magnetfeldgeneratoren besteht, mit welchen unter Verwendung einer geeigneten bekannten Software und Hardware eine genaue Positionierung zum Beispiel eines Katheters an einem Körper erfolgen kann.

Allgemein umfasst die erfindungsgemäße Vorrichtung Elemente, Vorrichtungen und Systeme, mit denen die oben beschriebenen Verfahrensschritte durchgeführt werden können.

Die Erfindung wird nachfolgend anhand bevorzugter Ausführungsbeispiele beschrieben. Dabei zeigen:
- Figur 1: schematisch ein Schaubild eines Verfahrens zur Planung und Durchführung einer Infusion;
- Figur 2: ein vereinfachtes Ablaufdiagramm einer erfindungsgemäß durchgeführten Infusion; und
- Figur 3: eine Vorrichtung, welche bei der Planung und Durchführung einer Infusion verwendet werden kann.

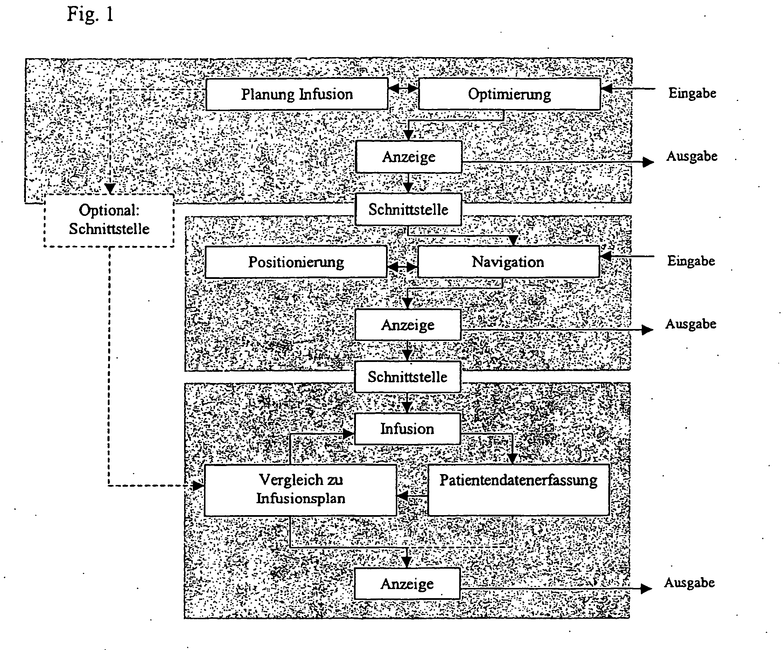

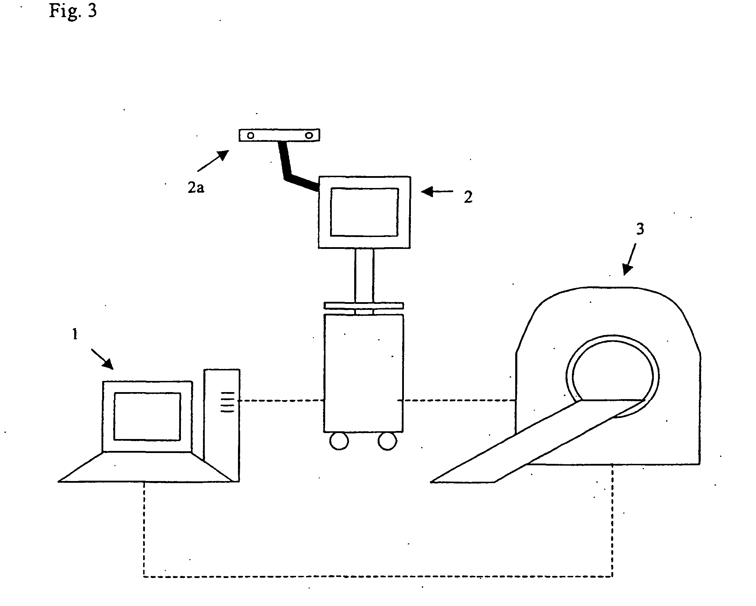

Figur 1 zeigt schematisch ein Ablaufdiagramm für die erfindungsgemäße Vorbereitung und Durchführung einer Infusion. Wie in Figur 1 gezeigt, werden Patientendaten zum Beispiel von einem Kernspintomographen eingegeben, welche verwendet werden, um einen bestimmten Gewebe-Zielbereich für die Infusion zu ermitteln und die zuzuführende Infusionsdosis zu planen. Diese Daten können zum Beispiel durch das Kemspinresonanz-System 3, wie in Figur 3 schematisch gezeigt, nach der Untersuchung eines zu behandelnden Patienten gewonnen werden. Unter Verwendung von zum Beispiel in Datenbanken abgespeicherten Parametern für die Eigenschaften der Gewebestrukturen und für verschiedene Kathetertypen kann nach der Ermittlung der genauen Lage des zu behandelnden Gewebevolumens ein oder mehrere für die Infusion geeignete Katheter ausgewählt werden. Die durch zum Beispiel das Kemspinresonanz-Verfahren gewonnenen Patientenparameter können zusammen mit den Katheterparametem und den zum Beispiel auch in Datenbanken gespeicherten Infusionsmittelparametern verwendet werden, um die Infusion zu planen. Dabei können die entsprechenden Parameter unter der Randbedingung optimiert werden, dass ein möglichst großer Anteil des Infusionsmittels mit einer gewünschten Konzentration in das Zielgewebe eingebracht wird, wobei möglichst wenig Infusionsmittel in ein außerhalb des Zielgewebes liegendes Gewebe gelangen soll. Allgemein sollten möglichst wenige Katheter oder Nadeln platziert werden, welche durch möglichst wenige Zugänge zugeführt werden. Diese optimierte Planung der Infusionsdosis wird über eine Anzeige ausgegeben, so dass zum Beispiel eine zweidimensionale oder dreidimensionale Darstellung durch Abbildung verschiedener Schnittebenen ausgegeben werden kann, um das Ergebnis der Infusionsplanung anzuzeigen.

Der so erstellte Infusionsplan wird über eine Schnittstelle an ein Navigationssystem übermittelt, wie beispielsweise das in Figur 3 schematisch gezeigte VectorVision®-System, um basierend auf den Planungsdaten den oder die ausgewählten Katheter an den vorgegebenen Stellen am Körper zu positionieren. Die Positionierung kann automatisch zum Beispiel unter Verwendung eines Roboters, oder durch das Navigationssystem geführt, von Hand erfolgen, wobei auf einer Anzeigevorrichtung dargestellt werden kann, ob ein Katheter richtig positioniert ist oder noch in eine bestimmte Richtung bewegt werden muss.

Nach erfolgter Positionierung des oder der Katheter(s) wird die eigentliche Infusion mit den durch die Planung vorgegebenen Infusionsmittelparametern durchgeführt. Dabei wird wiederum eine Patientendatenerfassung durchgeführt, um die tatsächliche Verteilung des Infusionsmittels im Gewebe zu ermitteln. Unter Verwendung der durch die Planung vorgegebenen Parameter und der darauf basierenden Simulationsergebnisse für die Infusion wird ein Vergleich zwischen tatsächlicher Infusionsmittelverteilung und vorausbestimmter gewünschter Infusionsmittelverteilung durchgeführt und gegebenenfalls werden die Parameter, wie zum Beispiel die Konzentration des Injektionsmittels, die Abgabemenge oder der Injektionsdruck zur Durchführung der Infusion bevorzugt unter Berücksichtigung von bekannten Wirkmechanismen abgeändert, um das gewünschte, geplante Infusionsergebnis zu erhalten. Wiederum kann die gemessene tatsächliche Verteilung der Infusionsmittel-Konzentration bevorzugt zusammen mit eventuellen Abweichungen und Korrekturverfahren über eine Anzeige ausgegeben werden, um es zum Beispiel einer Bedienperson zu ermöglich manuell in das Injektionsverfahren einzugreifen.

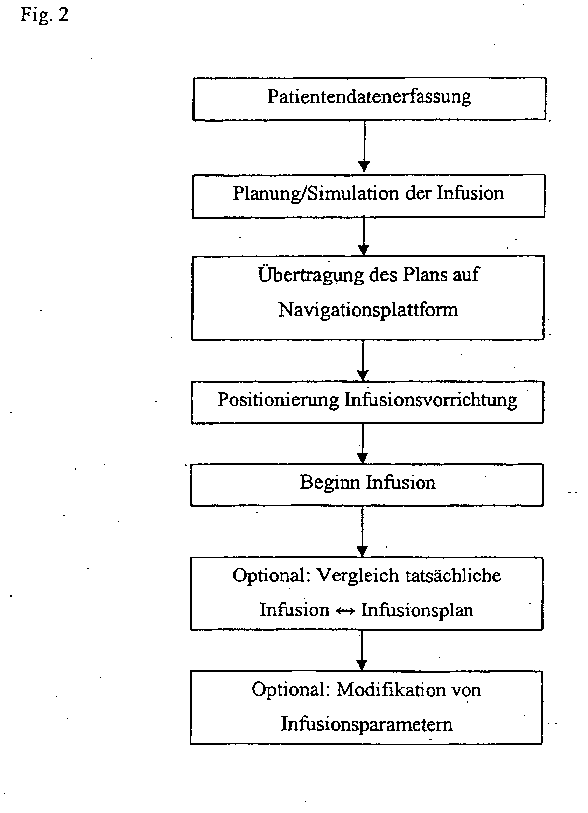

Figur 2 zeigt schematisch einen vereinfachten Ablauf einer erfindungsgemäßen Planung und Durchführung einer Injektion. Zunächst werden Patientendaten durch ein bildgebendes diagnostisches Verfahren wie beispielsweise ein Kernspinresonanz-Verfahren erfasst, um die aktuellen Patientenparameter wie zum Beispiel Gewebedichte, Durchblutung und Lage eines zu behandelnden Gewebes zu erhalten. Unter Verwendung der so ermittelten Patientenparameter sowie mit aus einer Datenbank erhaltenen und/oder für eine bestimmte Infusion vorgegebenen Katheter- und Infusionsmittelparametern wird die Infusion geplant und/oder simuliert. Basierend auf den so ermittelten Parameterdaten wird der Infusionsplan an eine Navigationsplattform weitergegeben, mit welcher der oder die Katheter wie im Infusionsplan vorgesehen am Patienten positioniert werden sollen. Die Infusion beginnt nach der Positionierung der Infusionsvorrichtung und wird mit den geplanten und gegebenenfalls simulierten Parametern durchgeführt, wobei optional, wie in Figur 1 und 2 gezeigt, ein Vergleich der tatsächlich durchgeführten Infusion mit dem Infusionsplan durchgeführt wird und bei Abweichungen eine Modifikation der entsprechenden Parameter bevorzugt unter Ausnutzung bekannter Wirkmechanismen vorgenommen wird.

Figur 3 zeigt schematisch eine Vorrichtung, welche zur Planung und Durchführung einer erfindungsgemäßen Infusion verwendet werden kann. Patientendaten werden in einem Kernspintomographen 3 erhalten und an ein Planungssystem 1 sowie ein Navigationssystem 2 weitergegeben. Mit dem Navigationssystem 2 werden unter Verwendung von zum Beispiel bekannten an einem oder mehreren Kathetern angebrachten Reflektoren oder Markern der oder die Katheter an einer gewünschten Stelle an einem Körper positioniert, wobei Positionsdaten der Marker durch IR-Kameras 2a erfasst werden. Das Planungssystem 1 ermittelt für eine vorgegebene durchzuführende Infusion unter Verwendung der vom Kemspinresonanz-system 3 ermittelten Patientenparameter die geeigneten Katheterparameter und Infusionsparameter, um die Infusion durchzuführen.

## Patentansprüche

1. Verfahren zur Planung einer Infusion, wobei Patientendaten erfasst werden und Informationen zur Gewebestruktur, Gewebedichte, Durchblutung und/oder Stoffwechseleigenschaften des Gewebes als Patientenparameter aus den erfassten Patientendaten gewonnen werden und die durchzuführende Infusion unter Verwendung dieser Patientenparameter geplant wird, wobei eine Simulation der Infusionsmittelverteilung basierend auf den Patientenparametern und Infusionsmittelparametern durchgeführt wird, **dadurch gekennzeichnet, daß** mindestens eine Infusionsvorrichtung unter Verwendung der erfassten Patientendaten bezüglich des Infusionsorts mittels eines Navigationssystems unter Verwendung von Markem an dem Körper positioniert wird.

2. Verfahren nach einem der vorhergehenden Ansprüche, wobei die Patientendaten durch ein Kernspinresonanzverfahren (MRI), ein Computertomographieverfahren (CT), ein Röntgenverfahren oder ein Ultraschallverfahren erfasst werden.

3. Verfahren nach einem der vorhergehenden Ansprüche, wobei Infusionsmittelparameter, welche chemische, biologische und/oder physikalische Eigenschaften des Infusionsmittels definieren, zur Planung der Infusion verwendet werden.

4. Verfahren nach einem der vorhergehenden Ansprüche, wobei Katheterparameter zur Planung der Infusion verwendet werden.

5. Verfahren nach einem der vorhergehenden Ansprüche, wobei eine Simulation der Infusionsmittelverteilung basierend auf den Patientenparametern, Katheterparametern und Infusionsmittelparametern durchgeführt wird.

6. Verfahren nach einem der vorhergehenden Ansprüche, wobei ein Zielvolumen und/oder eine Infusionsmittelverteilung im Patienten vorgegeben wird und basierend darauf hierfür erforderliche Katheterparameter und Infusionsmittelparameter ermittelt werden.

7. Vorrichtung zur Planung einer Infusion mit einem Patientendatenerfassungssystem (3) und einem Computersystem (1) zur Durchführung der Planung der Infusion basierend auf Informationen zur Gewebestruktur, Gewebedichte, Durchblutung und/oder Stoffwechseleigenschaften des Gewebes als Patientenparameter, welche aus den erfassten Patientendaten gewonnen werden, wobei eine Simulation der Infusionsmittelverteilung basierend auf den Patientenparametern und Infusionsmittelparametern durchgeführt werden kann, **dadurch gekennzeichnet, daß** die Vorrichtung ein Navigationssystem (2) zum Positi- <SHY>Navigationssystem (2) zum Positionieren mindestens eines Katheters basierend auf den Planungsdaten aufweist.

8. Vorrichtung zur Planung einer Infusion nach Anspruch 7 und zur Durchführung einer Infusion mit einer Verifikationsvorrichtung zum Vergleichen der geplanten Infusionsdaten mit tatsächlichen Infusionsdaten.

9. Verfahren zur Planung einer Infusion nach einem der Ansprüche 1 bis 6 und zum nachfolgenden Vergleichen der tatsächlichen Infusionsdaten mit den geplanten Infusionsdaten.

10. Verfahren nach Anspruch 9, wobei Abweichungen zwischen geplanten und tatsächlichen Infusionsdaten ermittelt werden.

11. Verfahren nach Anspruch 10, wobei basierend auf den ermittelten Abweichungen eine Korrektur der Infusionsmittelparameter vorgenommen wird.

## Claims

1. A method for planning an infusion, wherein patient data are captured and information on the tissue structure, tissue density, blood flow and/or metabolic properties of said tissue is obtained as patient parameters from said captured patient data and the infusion to be carried out is planned using said patient data, wherein the distribution of said infusing medium is simulated based on said patient parameters and parameters of said infusing medium, **characterised in that** at least one infusion device is positioned using said patient data, with respect to the infusion location, by means of a navigation system using markers on said body.

2. The method as set forth in any one of the preceding claims, wherein said patient data are captured by a magnetic resonance method (MRI), a computer tomography method (CT), an x-ray method or an ultrasound method.

3. The method as set forth in any one of the preceding claims, wherein parameters of said infusing medium, defining chemical, biological and/or physical properties of said infusing medium, are used for planning said infusion.

4. The method as set forth in any one of the preceding claims, wherein catheter parameters are used for planning said infusion.

5. The method as set forth in any one of the preceding claims, wherein the distribution of said infusing medium is simulated based on said patient parameters, catheter parameters and said parameters of said infusing medium.

6. The method as set forth in any one of the preceding claims, wherein a target volume and/or a distribution of infusing medium in the patient is pre-set, and the catheter parameters and parameters of said infusing medium required for this are determined on the basis of these.

7. A device for planning an infusion, comprising a patient data capturing system (3) and a computer system (1) for carrying out planning of the infusion, based on information on the tissue structure, tissue density, blood flow and/or metabolic properties of said tissue as patient parameters obtained from said captured patient, wherein the distribution of said infusing medium can be simulated based on said patient parameters and parameters of said infusing medium, **characterised in that** said device comprises a navigation system (2) for positioning at least one catheter, based on said planning data.

8. The device for planning an infusion as set forth in claim 7 and for carrying out an infusion, comprising a verification device for comparing the planned infusion data with actual infusion data.

9. The device for planning an infusion as set forth in any one of claims 1 to 6 and for then comparing the actual infusion data with the planned infusion data.

10. The method as set forth in claim 9, wherein deviations between said planned and said actual infusion data are determined.

11. The method as set forth in claim 10, wherein the parameters of said infusing medium are corrected, based on said determined deviations.

## Revendications

1. Procédé pour planifier une perfusion, les données du patient étant saisies et des informations sur la structure tissulaire, l'épaisseur tissulaire, la circulation sanguine et / ou les propriétés métaboliques du tissu, en tant que paramètres du patient, étant acquises à partir des données du patient saisies et la perfusion à effectuer étant planifiée en utilisant ces paramètres du patient, une simulation de la distribution des agents de perfusion étant réalisée en se fondant sur les paramètres du patient et les paramètres des agents de perfusion, **caractérisé en ce qu'**au moins un dispositif de perfusion est positionné en utilisant des marqueurs sur le corps humain, en utilisant les données du patient saisies, en ce qui concerne le lieu de la perfusion, au moyen d'un système de navigation.

2. Procédé selon la revendication précédente, les données du patient étant saisies par un procédé de résonance magnétique nucléaire (RMN), un procédé de tomodensitométrie (TD), un procédé radiographique ou un procédé d'examen aux ultrasons.

3. Procédé selon l'une des revendications précédentes, les paramètres des agents de perfusion, lesquels définissent les propriétés chimiques, biologiques et / ou physiques de l'agent de perfusion, étant utilisés pour planifier la perfusion.

4. Procédé selon l'une des revendications précédentes, les paramètres du cathéter étant utilisés pour planifier la perfusion.

5. Procédé selon l'une des revendications précédentes, une simulation de la distribution des agents de perfusion, se fondant sur les paramètres du patient, les paramètres du cathéter et les paramètres des agents de perfusion, étant réalisée.

6. Procédé selon l'une des revendications précédentes, un volume cible et / ou une distribution des agents de perfusion dans le corps du patient étant prédéterminée et les paramètres du cathéter et les paramètres des agents de perfusion, indispensables à cet effet, étant déterminés sur cette base.

7. Dispositif pour planifier une perfusion avec un système de saisie des données du patient (3) et un système informatique (1), pour réaliser la planification de la perfusion, se fondant sur des informations sur la structure tissulaire, l'épaisseur tissulaire, la circulation sanguine et / ou les propriétés métaboliques du tissu, en tant que paramètres du patient, lesquelles ont été acquises à partir des données du patient saisies, une simulation de la distribution des agents de perfusion pouvant être réalisée, en se fondant sur les paramètres du patient et les paramètres des agents de perfusion, **caractérisé en ce que** le dispositif présente un système de navigation (2), pour positionner au moins un cathéter sur la base des données de planification.

8. Dispositif pour planifier une perfusion et pour réaliser une perfusion avec un dispositif de vérification, pour comparer les données de perfusion planifiées avec des données de perfusion effectives.

9. Dispositif pour planifier une perfusion selon l'une des revendications 1 à 6 et pour comparer consécutivement les données de perfusion effectives avec les données de perfusion planifiées.

10. Procédé selon la revendication 9, des divergences entre données de perfusion planifiées et effectives étant déterminées.

11. Procédé selon la revendication 10, une correction des paramètres des agents de perfusion étant entreprise sur la base des divergences déterminées.
